# FASCICULE DE BREVET EUROPEEN

(11) **EP 3 482 774 B1**
(45) Date de publication et mention de la délivrance du brevet: **24.06.2020**
(21) Numéro de dépôt: 18197399.1
(22) Date de dépôt: 28.09.2018
(51) Int. Cl.: A61K 47/10, A61K 47/14, A61K 9/107, A61K 47/44, A61K 9/20

(54) **SYSTEME AUTO-EMULSIONNABLE SOLIDE ET SON PROCEDE DE FABRICATION PAR IMPRESSION EN 3 DIMENSIONS**
FESTES SELBST-EMULSIONIERBARES SYSTEM UND SEIN HERSTELLUNGSVERFAHREN MITHILFE VON 3D-DRUCK
SOLID SELF-EMULSIONABLE SYSTEM AND METHOD FOR MAKING SAME BY PRINTING IN 3 DIMENSIONS

(30) Priorité: 10.11.2017 FR 1760615
(43) Date de publication de la demande: 15.05.2019
(73) Titulaire: GATTEFOSSE SAS, 69800 Saint Priest (FR)
(72) Inventeur: JANNIN, Vincent, 69003 LYON (FR); BOYD, Benjamin, WARRANDYTE, Victoria 3113 (AU); VITHANI, Kapilkumar, 364760 GUJARAT (IN)
(74) Mandataire: Cabinet Laurent & Charras

(56) Documents cités:
- WO-A1-2016/192680
- WO-A2-03/003999
- FR-A1- 2 827 770

## Description

### DOMAINE TECHNIQUE

L'invention concerne la composition d'un système comprenant au moins un actif, auto-émulsionnable au contact d'une phase hydrophile apportée notamment, après ingestion, par le liquide physiologique, qui se présente sous forme solide, sans recourir à l'utilisation d'une tunique ou d'un additif conférant sa solidité au système. Un tel système est obtenu par impression en 3 dimensions, permettant de produire une forme pharmaceutique solide dont la détermination des paramètres de forme permet un contrôle de libération de l'actif.

### ETAT DE LA TECHNIQUE

Actuellement, la majorité des nouvelles molécules pharmaceutiques découvertes sont lipophiles et possèdent une faible solubilité dans l'eau, ce qui conduit à une faible biodisponibilité, la production de variations intra- et interindividuelles et une absence d'effet proportionnel des concentrations d'actif pharmaceutique.

Le faible pouvoir soluble dans l'eau de ces molécules actives est un enjeu pour le développement de compositions orales solides et efficaces, notamment en termes de formulation, de biodisponibilité, de contrôle de la libération de l'actif et de commercialisation de nouveaux produits pharmaceutiques.

Une autre méthode d'administration d'un actif pharmaceutique, peu soluble dans l'eau, consiste à inclure cet actif dans la phase huileuse d'un système émulsionnable.

A titre d'exemple, on peut citer les systèmes auto-émulsionnables, les systèmes auto-microémulsionnables, les émulsions, les solutions micellaires ou encore les liposomes.

Le système auto-émulsionnable (SEDDS ou *self-emulsifying drug delivery system*) et le système auto-microémulsionnable (SMEDDS ou *self-microemulsifying drug delivery system*) sont composés d'un ou plusieurs excipients lipidiques, d'un ou plusieurs tensioactifs (ou surfactants) et éventuellement d'un co-tensioactif (ou co-surfactant) et/ou d'un co-solvant. Lorsqu'ils sont introduits en milieu aqueux, sous agitation légère, ces systèmes sont capables de former une émulsion fine de type huile dans eau (H/E), une microémulsion ou une solution micellaire.

Par rapport à la composition d'un système auto-émulsionnable, la composition d'un système auto micro-émulsionnable diffère en ce qu'elle comprend en plus d'une phase lipidique et d'une phase tensioactive, au moins un agent co-tensioactif.

Ces systèmes ont montré un intérêt tout particulier dans l'administration par voie orale des principes actifs peu solubles dans l'eau, le milieu stomacal jouant alors le rôle de la phase dispersante aqueuse. Il apparaît que l'incorporation de ces principes actifs dans ces systèmes auto-émulsionnables permet d'améliorer les facteurs limitant l'absorption de ce type d'actif tels que sa faible solubilité dans l'eau ou sa cinétique de dissolution. De tels systèmes sont notamment décrits dans le document du Demandeur EP 0 670 715 B1.

Lors de l'administration d'un tel système, au contact d'une phase hydrophile apportée après ingestion par le liquide physiologique, la motilité gastrique entraîne une agitation du contenu stomacal et permet donc l'auto-émulsification qui conduit à la formation d'une dispersion très fine de gouttelettes de lipides, soit un colloïde. Le principe actif de nature lipophile se retrouve emprisonné dans les gouttelettes d'huile. La mise à disposition du principe actif sous forme de gouttelettes, dont la taille est généralement inférieure à 300 nm, crée ainsi une large distribution de la substance active tout le long du tractus gastro-intestinal favorisant sa dissolution et son absorption.

Les travaux de Pouton (Eur J Pharm Sci 2006 ; 29 :278-87) ont permis de mettre en place un système de classification de ces formulations lipidiques (LFCS ou *Lipid formulation classification system*), dans le but d'harmoniser leur compréhension. Ainsi, selon le LFCS, les formulations lipidiques peuvent être classées dans quatre catégories différentes : types I, II, III (A et B) et IV. Les formulations SEDDS correspondent au type II et les formulations SMEDDS correspondent aux types IIIA et IIIB selon le LFCS. Elles ont pour avantages d'augmenter la biodisponibilité de l'actif, de diminuer les variations interindividuelles et de permettre une absorption de l'actif indépendante de la digestion et donc des acides biliaires.

Les systèmes auto-émulsionnables se distinguent des émulsions et des solutions micellaires notamment de par la taille des gouttelettes dont elles sont constituées. En effet, la taille des gouttelettes d'une auto-émulsion est comprise entre 250 nanomètres (nm) et 2 micromètres (µm), et celle des gouttelettes d'une auto-microémulsion est inférieure à 250 nm.

En résumé, la formulation auto-émulsionnable correspond donc à un mélange comprenant une phase huileuse, au moins un tensioactif et éventuellement au moins un agent co-tensioactif, dans des proportions appropriées pour former rapidement une émulsion ou microémulsion H/E, grâce à une motilité gastro-intestinale modérée, lorsque la formulation est exposée au milieu aqueux du tractus gastro-intestinal.

En pratique, la formation rapide d'une auto-émulsion ou auto-microémulsion permet de conserver l'actif pharmaceutique sous forme dissoute. De même, la faible taille des gouttelettes offre une interface importante, ce qui accélère le taux d'absorption des molécules ayant une faible solubilité.

De plus, la fraction lipidique de la formulation auto-émulsionnable favorise la recapture de l'actif lipophile et liposoluble par le système lymphatique intestinal, ce qui évite la biotransformation systémique et la clairance de cet actif.

Ainsi, de par la formation de l'émulsion *in situ*, les systèmes auto-émulsionnables permettent de solubiliser le principe actif et par conséquent augmenter sa biodisponibilité. Ces caractéristiques font de la formulation auto-émulsionnable un choix pertinent pour l'administration orale d'actif pharmaceutique lipophile et peu biodisponible.

Les compositions auto-émulsionnables peuvent se présenter, à température ambiante, sous forme semi-solide/pâteuse ou liquide, en fonction de la nature et de la proportion même des corps gras qui les composent.

Cependant, la nature liquide ou semi-solide/pâteuse des systèmes auto-émulsionnables conduit à réduire leur commercialisation du fait de difficultés de production, de manipulation, de stockage, de fourniture ou encore de stabilité.

En pratique, des systèmes auto-émulsionnables liquides sont coulés en gélules molles ou gélules dures. Néanmoins, un tel conditionnement pour administration orale n'est pas toujours bien accepté par les patients.

Une autre limite liée à la présentation sous forme de gélule se trouve être sa forme monolithique. Dans le cas notamment où le mélange n'est pas totalement liquide mais présente une viscosité assez élevée, voire semi-solide/pâteuse. Ce type de formule, au contact de l'eau, peut mettre un certain temps à se déliter et donc à former l'émulsion fine, retardant alors la mise à disposition du principe actif.

Dans le cas des formulations liquides à température ambiante, le principe actif devra être totalement solubilisé afin d'éviter tout problème de déphasage ou de sédimentation dans la forme finale. Cette solubilisation totale n'est pas toujours facile à obtenir car des problèmes de sursaturation peuvent conduire à une reprécipitation du principe actif au cours du temps. De plus, il est parfois très difficile de trouver des excipients présentant une bonne capacité de solubilisation correspondant à la dose de principe actif qu'il faut incorporer dans la formule.

Alternativement, une composition auto-émulsionnable sous forme liquide peut être chargée sur un vecteur solide tel que les dérivés de silicate, le dextran ou le dioxyde de silicone, pour produire un comprimé.

Toutefois, une large quantité de vecteur solide est nécessaire pour adsorber la formulation liquide, ce qui conduit à générer une forme finale du comprimé de grand volume et donc difficilement ingérable. De plus, la reproductibilité de la charge de l'actif n'est pas toujours optimale et le temps de production est relativement long.

En parallèle, l'impression en 3 dimensions (3D) est une nouvelle technologie qui permet de révolutionner le monde médical. En effet, cette méthode est explorée en médecine pour imprimer, ou bio-imprimer, des tissus cardiaque ou hépatique à transplanter, pour imprimer des modèles de tumeur afin que les praticiens puissent s'entrainer avant une opération ; ou encore, par les dentistes pour l'impression de prothèse dentaire.

Récemment, l'impression en 3D a été utilisée pour imprimer un médicament antiépileptique, le lévétiracétam, commercialisé aux Etats-Unis sous la dénomination Spritam ®.

Ainsi, le problème que se propose de résoudre la présente invention est de fournir un système simple, auto-émulsionnable comprenant au moins un actif pharmaceutique, qui se présente sous forme solide et qui soit susceptible d'être obtenu selon un procédé simple, rapide, et conférant une très bonne reproductibilité. De manière générale, la présente invention vise un système auto-émulsionnable qui ne présente pas les inconvénients mentionnés précédemment.

### DESCRIPTION DE L'INVENTION

De manière inattendue, le Demandeur a constaté qu'il était possible de fabriquer des systèmes auto-émulsionnables solides par impression en 3 dimensions.

Plus précisément, le Demandeur a mis au point une nouvelle formulation simple, auto-émulsionnable, solide permettant avantageusement d'obtenir une forme pharmaceutique solide par impression en 3D. Cette forme pharmaceutique solide possède les caractéristiques d'un système auto-émulsionnable et permet donc d'augmenter la biodisponibilité d'au moins un actif pharmaceutique. Le paramétrage des caractéristiques de forme, de volume et de porosité de la forme pharmaceutique solide, issue du procédé selon la présente invention, permet de contrôler et d'optimiser la libération et l'absorption par le tractus gastro-intestinal d'au moins un actif après ingestion.

Ainsi, la présente invention a pour objet un système solide auto-émulsionnable au contact d'une phase hydrophile apportée, après ingestion, par le liquide physiologique, ledit système auto-émulsionnable comprenant:
- au moins un actif ;
et les excipients suivants :
- une phase lipophile ;
- un agent tensioactif présentant une balance hydrophile/lipophile (HLB) supérieure à 8 ;
- éventuellement un agent co-tensioactif ;
ledit système étant susceptible d'être obtenu par impression en 3 dimensions (3D).

Par « solide » selon la présente description, on entend une forme solide à température ambiante (environ 25°C).

Selon un mode de réalisation particulier, l'agent tensioactif du système auto-émulsionnable représente au moins 40% en poids total des excipients, de préférence 50%. Pour une valeur inférieure à 40% en poids total des excipients, le Demandeur a constaté qu'il n'était pas possible d'obtenir un système auto-émulsionnable sous forme solide, sans utilisation de tuniques ou d'additifs inducteurs de phase solide.

Selon un mode de réalisation particulier, l'agent tensioactif est solide à température ambiante et possède un point de fusion compris entre 25°C et 60°C.

Selon une caractéristique particulière de l'invention, l'agent tensioactif compris dans le système auto-émulsionnable est choisi dans le groupe comprenant esters de polyéthylène glycol, poloxamères, huiles éthoxylées, vitamine E éthoxylée, esters de sucre avec des acides gras, et leur mélange.

Avantageusement, l'ester de polyéthylène glycol est choisi dans le groupe comprenant du polyéthylène glycol-32 stéarate avantageusement Gélucire ® 48/16 et des polyoxylglycérides avantageusement Gélucire ® 44/14 ou Gélucire ® 50/13, et/ou ; le poloxamère est choisi dans le groupe comprenant poloxamère P188 et poloxamère P407, et/ou ; l'huile éthoxylée est le polyéthylène glycol-40 huile de ricin hydrogenée, et/ou ; la vitamine E éthoxylée est la vitamine E polyéthylène glycol succinate, et/ou ; l'ester de sucre avec des acides gras est choisi dans le groupe comprenant sucrose stéarate et sorbitan monoester.

Selon un mode de réalisation particulier, la phase lipophile constitutive du système auto-émulsionnable représente entre 5% et 60% en poids total des excipients, de préférence 50%.

Pour une valeur inférieure à 5% en poids total des excipients, le Demandeur a constaté que la quantité de phase lipophile est insuffisante pour former une émulsion ou une microémulsion après dilution du SEDDS / SMEDDS dans l'eau. Dans ce cas il s'agira d'une dissolution micellaire de l'huile par les agents tensioactifs. Par ailleurs, pour une valeur supérieure à 60% en poids total des excipients, le Demandeur a constaté que les agents tensioactifs peuvent difficilement émulsionner efficacement les corps gras solides. Selon une caractéristique, la phase lipophile est solide à température ambiante et possède un point de fusion comprise entre 25°C et 75°C.

Selon une caractéristique particulière de l'invention la phase lipophile du système auto-émulsionnable comprend au moins un composé choisi dans le groupe comprenant huiles végétales, triglycérides, diglycérides, monoglycérides, polyoxylglycérides, et leurs mélanges.

En tant que principe actif, peut être mis en œuvre tout type de principe actif, mais plus spécifiquement les principes actifs peu solubles dans l'eau présentant un Log P compris entre 1,5 et 8, à savoir par exemple : piroxicam, ketoconazole, miconazole, cinnarizine, griseofulvine, itraconazole, fenofibrate, acide mefenamique, etc.

Le principe actif pourra être soluble partiellement ou totalement dans les excipients lipidiques. Les principes actifs non solubles dans les excipients mais présentant une bonne affinité avec les lipides utilisés pourront être également mis en œuvre.

Selon l'invention, l'actif correspond à un composé lipophile et possédant une faible solubilité dans l'eau, ce qui résulte en une faible biodisponibilité. Avantageusement l'actif est choisi parmi le groupe constitué de la cinnarizine et le fenofibrate. Ces molécules sont connues pour être peu biodisponibles du fait de leur caractère lipophile et de leur faible solubilité dans l'eau.

Selon un mode de réalisation particulier, la formulation selon l'invention comprend en outre, un co-solvant, avantageusement choisi parmi le groupe constitué de l'éthanol et du Transcutol®.

En pratique, le co-solvant permet d'améliorer la solubilisation de l'actif.

De manière générale, un même excipient, en fonction de sa composition, peut remplir à la fois le rôle de tensioactif et de phase lipophile, ou peut être en combinaison avec un autre excipient pour l'une ou l'autre de ces deux fonctions.

Selon un autre mode de réalisation particulier, le système selon l'invention est constitué de Gélucire ® 44/14 ou de Gélucire ® 50/13. Ainsi, la fraction lipidique du Gélucire ® 44/14 ou du Gélucire ® 50/13 constitue la phase lipophile, et la fraction tensioactive de ces composés respectifs constitue la phase tensioactive du système solide auto-émulsionnable.

La présente invention concerne également le procédé de fabrication d'un système solide auto-émulsionnable au contact d'une phase hydrophile apportée, après ingestion, par le liquide physiologique, ledit système auto-émulsionnable comprenant:
- au moins un actif ;
et les excipients suivants :
- une phase lipophile ;
- un agent tensioactif présentant une balance hydrophile/lipophile (HLB) supérieure à 8 ;
- éventuellement un agent co-tensioactif ;
caractérisé en ce qu'il est mis en œuvre par impression en 3D,
consistant à :
- mélanger et chauffer le au moins un actif et les excipients à une température T1, avantageusement supérieure au point de fusion de l'excipient possédant le point de fusion maximal ;
- diminuer la température du mélange à une température T2, avantageusement la température ambiante ;
- augmenter la température pour atteindre une température T3 supérieure à la température de fusion du mélange des excipients, de préférence au moins égale à 15°C, avantageusement égale à 15°C, au-dessus de la température de fusion du mélange des excipients.

La température T1 selon l'invention correspond à une température supérieure au point de fusion de l'excipient présent dans le mélange possédant le point de fusion maximum.

La température T2 selon l'invention correspond à une température inférieure au point de fusion de l'excipient présent dans le mélange possédant le point de fusion maximum.

Avantageusement la température T2 est la température ambiante (environ 25°C).

La température T3 selon l'invention correspond à une température permettant l'obtention d'un mélange liquide, et facilement imprimable par impression en 3D.

Avantageusement la température T3 est de préférence au moins égale à 15°C, avantageusement égale à 15°C, au-dessus de la température de fusion du mélange des excipients de façon à s'assurer que le mélange reste liquide dans la buse d'impression. Cette température ne doit pas être trop élevée pour faciliter la cristallisation des couches de formules et ainsi contrôler la porosité de la forme pharmaceutique.

Selon le procédé mis en œuvre par la présente invention, l'imprimante en 3D imprime successivement de fines couches de système solide auto-émulsionnable. Ces couches sont refroidies immédiatement permettant la fabrication d'une forme pharmaceutique solide sans l'utilisation de tuniques ou d'additifs inducteurs de phase solide.

Avantageusement, un flux d'eau froide circule à travers le plateau de l'imprimante en 3D, grâce à une pompe péristaltique pour créer un environnement froid permettant une solidification rapide des couches imprimées.

Selon un mode de réalisation particulier de l'invention, préalablement au mélange d'au moins un actif ; une phase lipophile ; un agent tensioactif présentant une balance hydrophile/lipophile (HLB) supérieure à 8 ; éventuellement un agent co-tensioactif, on détermine au moyen d'un logiciel adapté les paramètres de forme, ratio surface/volume et porosité du système solide auto-émulsionnable souhaité susceptible d'être obtenu par impression en 3D.

Avantageusement, un tel procédé permet de modifier les propriétés de libération de l'actif dudit système par le choix des paramètres mentionnés précédemment pour l'impression en 3D.

Par exemple, et de façon non limitative, le système obtenu sous forme solide, par impression en 3D selon le procédé de l'invention, peut être sous forme de cylindre, d'anneau, de cube, de pyramide ou de triangle. La définition de la forme du système, telle qu'elle ne peut pas être obtenue par des méthodes de solidification traditionnelle, permet de corréler les paramètres géométriques avec une cinétique de dispersion et de digestion du comprimé et donc de l'actif.

Par ailleurs, le procédé de fabrication du système selon l'invention permet également de contrôler la porosité du système par sélection de la densité de remplissage et de la taille des pores dans toute ou partie des couches du système auto-émulsionnable composant le système.

Un autre avantage selon le procédé de l'invention est la constance du poids du système, de préférence par l'utilisation de l'impression en 3D, dite par modélisation par dépôt fondu (FDM ou *fused deposition modeling*).

En résumé, le procédé selon l'invention permet de contrôler le nombre de couches imprimées, la longueur, la largeur, la densité de remplissage et le poids, et le temps d'impression de chaque couche. Ce contrôle précis des paramètres permet d'obtenir un taux de reproductibilité très élevé.

A cet avantage s'ajoute une diminution du coût et du temps de production. En effet, un système alimenté par de l'eau froide circule à travers de plateau de production via une pompe péristaltique pour créer un environnement froid et ainsi permettre une solidification rapide des couches imprimées de la forme pharmaceutique solide, de préférence inférieure à 5 minutes.

De plus, cette méthode permet de s'affranchir de l'utilisation des gélules, des tuniques, des vecteurs solides ou encore d'additifs inducteurs de phase solide qui présentent de nombreux inconvénients identifiés par l'état de la technique.

### LEGENDE DES FIGURES

**Figure 1**: Représentation en 3D de géométries sélectionnées du système auto-émulsionnable imprimé par impression en 3D (de gauche à droite : cylindre, triangle, anneau et cube).
**Figure 2** : Photographie en vue du dessus des géométries du système auto-émulsionnable obtenues par impression en 3D.
**Figure 3** : Photographie en vue latérale des géométries du système auto-émulsionnable obtenues par impression en 3D.

La manière dont l'invention peut être réalisée et les avantages qui en découlent, ressortiront mieux des exemples de réalisation qui suivent et des figures annexées, donnés à titre indicatif et non limitatif.

### EXEMPLES DE REALISATION

### 1- Composition d'un système auto-émulsionnable comprenant de la Cinnarizine

**Tableau 1 : Système selon l'invention comprenant comme actif la Cinnarizine**

| Produit | Pourcentage en poids de la composition |
|---|---|
| Cinnarizine | 6,97 |
| Gélucire ® 44/14 | 46,51 |
| Gélucire ® 48/16 | 23,26 |
| Kolliphor ® P 188 | 23,26 |
| Total | 100,00 |

### 2- Composition d'un système auto-émulsionnable comprenant du Fénofibrate

**Tableau 2 : Système selon l'invention comprenant comme actif le Fénofibrate**

| Produit | Pourcentage en poids de la composition |
|---|---|
| Fénofibrate | 6,97 |
| Gélucire ® 44/14 | 46,51 |
| Gélucire ® 48/16 | 23,26 |
| Kolliphor ® P 188 | 23,26 |
| Total | 100,00 |

### 3- Procédé de production de système auto-émulsionnable

Les composés selon l'exemple 1 ou 2, sont mélangés dans un bécher en verre et placés sur une plaque chauffante. La composition est chauffée à une température T1, supérieure au point de fusion de l'excipient présent dans la composition possédant le point de fusion maximal, pendant 30 minutes pour assurer le complet mélange des excipients. Selon ce mode de réalisation particulier, la température T1 est de 120°C pour la Cinnarizine et 80°C pour le Fénofibrate.

Ensuite, le mélange liquide est transféré dans une seringue extrudeuse en acier inoxydable KRA-15 pour permettre une solidification à la température T2, avantageusement la température ambiante (environ 25°C), et ainsi éviter la formation de bulles.

Après 15 minutes de solidification, la température de la seringue est augmentée à une température T3 qui s'élève à 15°C au-dessus de la température de fusion du mélange des excipients (50°C), pour faciliter sa cristallisation après dépôt en couche par le procédé d'impression en trois dimensions. Selon cet exemple de réalisation particulier, la température T3 correspond à 65°C. Selon l'invention, cette température correspond à une température permettant l'obtention d'un mélange imprimable par impression en 3D. La température est surveillée régulièrement pour éviter un écoulement continu ou une solidification de la composition dans la seringue.

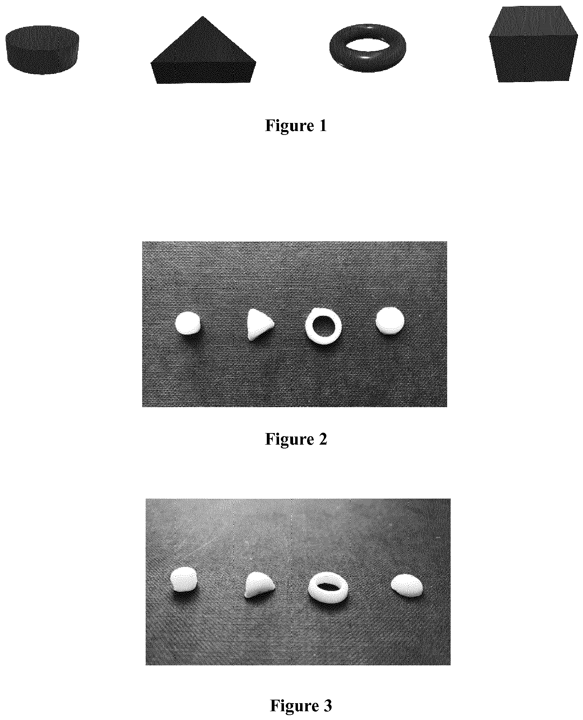

Après 10 minutes d'équilibre thermique, le mélange est imprimé en formes différentes, selon les modèles illustrés par la Figure 1. Pour ce qui est de l'impression, elle requiert l'utilisation d'une seringue d'impression en 3D pour les modèles d'imprimante par dépôt fondu (FDM) HYDREL SYSTEM 30M (HYDREL, Norcross, USA), en mettant en œuvre une taille de buse de seringue de 1,5 mm.

Les modèles de forme utilisés pour imprimer les comprimés sont conçus via le logiciel autoDesk 123D (Autodesk Inc. USA) et exportés en fichier de stéréolithographie (.stl).

La seringue de l'imprimante en 3D HYDREL SYSTEM 30M est calibrée dans les directions XYZ. Les paramètres d'impression sont les suivants : température d'impression de la buse à 65°C; vitesse de balayage de la buse pendant impression de 3mm/s ; hauteur de la couche imprimée de 0,4 mm; vitesse du ventilateur de refroidissement à 25% et la vitesse de conduction du piston à 1100 impulsion/s. La densité de remplissage est définie à 35% et le remplissage est concentrique.

Les géométries sélectionnées sont le cylindre, le triangle, l'anneau et le cube (Figure 1) et la taille des modèles est adaptée en utilisant une fonction échelle du logiciel pour obtenir un poids constant du système auto-émulsionnable de 215 mg.

Un flux d'eau froide circule à travers le plateau grâce à une pompe péristaltique pour créer un environnement froid permettant une solidification rapide des couches imprimées en un temps inférieur à 5 minutes.

La longueur (L), la largeur (1), et la hauteur (h) de chaque modèle, le nombre de couches à imprimer et le temps d'impression sont présentés dans le tableau 3.

**Tableau 3 : Paramètres d'impression**

| **Actif** | **Forme** | **L x 1 x h (mm)** | **Nombre de couches imprimées** | **Temps d'impression (min)** |
|---|---|---|---|---|
| Cinnarizine | Cylindre | 11x11x3,3 | 9 | 3,16 |
| Cinnarizine | Triangle | 12,3 x 12,3 x 3,8 | 11 | 3,26 |
| Cinnarizine | Anneau | 13,75 x 13,75 x 3,4 | 9 | 3,13 |
| Cinnarizine | Cube | 7,7 x 7,3 x 5,5 | 15 | 3,07 |
| Fénofibrate | Cylindre | 9,6 x 9,6 x 2,75 | 8 | 2,20 |
| Fénofibrate | Triangle | 12 x 12 x 3,5 | 9 | 2,33 |
| Fénofibrate | Anneau | 12,3 x 12,3 x 3,4 | 9 | 2,36 |
| Fénofibrate | Cube | 7 x 7 x 4,5 | 12 | 2,24 |

Les comprimés obtenus selon le procédé d'impression décrit précédemment sont illustrés par les Figures 2 et 3.

## Revendications

1. Système solide auto-émulsionnable au contact d'une phase hydrophile apportée, après ingestion, par le liquide physiologique, ledit système auto-émulsionnable comprenant:
- au moins un actif ;
et les excipients suivants :
- une phase lipophile ;
- un agent tensioactif présentant une balance hydrophile/lipophile (HLB) supérieure à 8;
- éventuellement un agent co-tensioactif ;
ledit système étant susceptible d'être obtenu par impression en 3 dimensions (3D).

2. Système selon la revendication 1, **caractérisé en ce que** l'agent tensioactif représente au moins 40% en poids total des excipients, de préférence 50%.

3. Système selon l'une des revendications précédentes, **caractérisé en ce que** l'agent tensioactif est solide à température ambiante et possède un point de fusion compris entre 25°C et 60°C.

4. Système selon l'une des revendications précédentes, **caractérisé en ce que** l'agent tensioactif est choisi dans le groupe comprenant esters de polyéthylène glycol, poloxamères, huiles éthoxylées, vitamine E éthoxylée, esters de sucre avec des acides gras, et leur mélange.

5. Système selon la revendication 4, **caractérisé en ce que** :
- l'ester de polyéthylène glycol est choisi dans le groupe comprenant du polyéthylène glycol-32 stéarate avantageusement Gélucire ® 48/16 et des polyoxylglycérides avantageusement Gélucire ® 44/14 ou Gélucire ® 50/13, et/ou ;
- poloxamère est choisi dans le groupe comprenant poloxamère P188 et poloxamère P407, et/ou ;
- l'huile éthoxylée est le polyéthylène glycol-40 huile de ricin hydrogenée, et/ou ;
- la vitamine E éthoxylée est la vitamine E poléthylène glycol succinate, et/ou ;
- l'ester de sucre avec des acides gras est choisi dans le groupe comprenant sucrose stéarate et sorbitan monoester.

6. Système selon l'une des revendications précédentes, **caractérisé en ce que** la phase lipophile représente entre 5% et 60% en poids total des excipients, de préférence 50%.

7. Système selon l'une des revendications précédentes, **caractérisé en ce que** la phase lipophile est solide à température ambiante et possède un point de fusion compris entre 25°C et 75°C.

8. Système selon l'une des revendications précédentes, **caractérisé en ce que** la phase lipophile comprend au moins un composé choisi dans le groupe comprenant huiles végétales, triglycérides, diglycérides, monoglycérides, polyoxylglycérides, et leurs mélanges.

9. Système selon l'une des revendications précédentes, **caractérisé en ce qu'**il comprend en outre, un co-solvant avantageusement choisi dans le groupe comprenant l'éthanol et le Transcutol®.

10. Procédé de fabrication d'un système solide auto-émulsionnable au contact d'une phase hydrophile apportée, après ingestion, par le liquide physiologique, ledit système auto-émulsionnable comprenant:
- au moins un actif ;
et les excipients suivants :
- une phase lipophile ;
- un agent tensioactif présentant une balance hydrophile/lipophile (HLB) supérieure à 8 ;
- éventuellement un agent co-tensioactif ;
**caractérisé en ce qu'**il est mis en œuvre par impression en 3D.

11. Procédé selon la revendication 10, **caractérisé en ce qu'**il consiste à :
- mélanger et chauffer le au moins un actif et les excipients à une température T1, avantageusement supérieure au point de fusion de l'excipient possédant le point de fusion maximal ;
- diminuer la température du mélange des excipients à une température T2, avantageusement la température ambiante ;
- augmenter la température pour atteindre une température T3 supérieure à la température de fusion du mélange des excipients, de préférence au moins égale à 15°C, avantageusement égale à 15°C, au-dessus du point de fusion du mélange des excipients.

12. Procédé selon l'une des revendications 10 ou 11, **caractérisé en ce que** préalablement au mélange d'au moins un actif ; une phase lipophile ; un agent tensioactif présentant une balance hydrophile/lipophile (HLB) supérieure à 8 ; éventuellement un agent co-tensioactif, on détermine au moyen d'un logiciel adapté les paramètres de forme, ratio surface/volume et porosité du système solide auto-émulsionnable souhaité susceptible d'être obtenu par impression en 3D.

## Patentansprüche

1. Festes, selbst- emulsionierbares System in Kontakt mit einer hydrophilen Phase, die nach Einnahme durch die physiologische Flüssigkeit bereitgestellt wird, wobei das selbst- emulsionierbare System umfasst:
- mindestens einen Wirkstoff;
sowie die folgenden Hilfsstoffe:
- eine lipophile Phase;
- ein Tensid mit einem hydrophilen/ lipophilen Gleichgewicht (HLB) von mehr als 8;
- gegebenenfalls ein Co- Tensid;
dabei kann dieses System durch Drucken in 3 Dimensionen (3D) erhalten werden.

2. System nach Anspruch 1, **dadurch gekennzeichnet, dass** das Tensid mindestens 40% des Gesamtgewichtes der Hilfsstoffe bildet, vorzugsweise 50%.

3. System nach einem der vorhergehenden Ansprüche, **dadurch gekennzeichnet, dass** das Tensid bei Raumtemperatur fest ist und einen Schmelzpunkt zwischen 25°C und 60°C hat.

4. System nach einem der vorhergehenden Ansprüche, **dadurch gekennzeichnet, dass** das Tensid ausgewählt wird aus der Gruppe mit Polyethylenglycol- Estern, Poloxameren, ethoxylierten Ölen, ethoxyliertem Vitamin E, Zuckerester aus Fettsäuren und ihren Mischungen.

5. System nach Anspruch 4, **dadurch gekennzeichnet, dass**:
- der Polyethylenglycol- Ester ausgewählt wird aus der Gruppe mit Polyethylenglycol-32- Stearat, vorzugsweise Gélucire® 48/16 und Polyoxylglyzeriden vorzugsweise Gélucire® 44/14 oder Gélucire® 50/13, und/oder
- das Poloxamer ausgewählt wird aus der Gruppe mit Poloxamer P188 und Poloxamer P407, und/oder;
- es sich bei dem ethoxylierten Öl um Polyethylenglycol-40 gehärtetes Rizinöl, und/oder handelt;
- es sich bei dem ethoxylierten Vitamin E, um ethoxyliertes Vitamin E-Polyethylenglycol- Succinat handelt, und/oder;
- der Zuckerester aus Fettsäuren ausgewählt wird aus der Gruppe mit Sucrosestearat und Sorbitan- Monoester.

6. System nach einem der vorhergehenden Ansprüche, **dadurch gekennzeichnet, dass** die lipophile Phase zwischen 5% und 60% des Gesamtgewichtes der Hilfsstoffe bildet, vorzugsweise 50%.

7. System nach einem der vorhergehenden Ansprüche, **dadurch gekennzeichnet, dass** die lipophile Phase bei Raumtemperatur fest ist und einen Schmelzpunkt zwischen 25°C und 75°C hat.

8. System nach einem der vorhergehenden Ansprüche, **dadurch gekennzeichnet, dass** die lipophile Phase mindestens eine Verbindung enthält, die ausgewählt wird aus der Gruppe mit Pflanzenölen, Triglyceriden, Diglyceriden, Monoglyceriden, Polyoxylglyceriden, und ihren Mischungen.

9. System nach einem der vorhergehenden Ansprüche, **dadurch gekennzeichnet, dass** es außerdem ein Co- Tensid enthält, vorteilhafterweise ausgewählt aus der Gruppe mit Ethanol und Transcutol®.

10. Verfahren zur Herstellung eines festen, selbst- emulsionierbaren Systems in Kontakt mit einer hydrophilen Phase, die nach Einnahme durch die physiologische Flüssigkeit bereitgestellt wird, wobei das selbst- emulsionierbare System umfasst:
- mindestens einen Wirkstoff;
sowie die folgenden Hilfsstoffe:
- eine lipophile Phase;
- ein Tensid mit einem hydrophilen/ lipophilen Gleichgewicht (HLB) von mehr als 8;
- gegebenenfalls ein Co- Tensid;
**dadurch gekennzeichnet, dass** es durch 3D- Druck eingesetzt wird.

11. Verfahren nach Anspruch 10, **dadurch gekennzeichnet, dass** es in folgenden Schritten besteht:
- Mischen und erwärmen des mindestens einen Wirkstoffs und der Hilfsstoffe auf eine Temperatur Tl, die vorteilhafter über dem Schmelzpunkt des Hilfsstoffs liegen sollte der den höchsten Schmelzpunkt aufweist;
- Verringerung der Temperatur der Hilfsstoffe auf eine Temperatur T2, vorteilhafterweise die Raumtemperatur;
- - Erhöhen der Temperatur um eine Temperatur T3 zu erreichen, die höher liegt als die Schmelztemperatur der Hilfsstoffmischung, vorzugsweise mindestens gleich 15°C, vorteilhafterweise gleich 15°C, über dem Schmelzpunkt der Hilfsstoffmischung.

12. Verfahren nach einem der Ansprüche 10 oder 11, **dadurch gekennzeichnet, dass** vor der Mischung mindestens eines Wirkstoffs, einer lipophilen Phase; eines Tensids mit einem hydrophilen/ lipophilen Gleichgewicht (HLB) von mehr als 8; gegebenenfalls eines Co- Tensids, mittels einer geeigneten Software die Parameter von Form, Flächen/ Volumenverhältnis und Porosität des gewünschten selbstemulsionierbaren Systems bestimmt werden, das durch 3D- Druck erhalten werden kann.

## Claims

1. Solid self-emulsionable system in contact with a hydrophilic phase carried, after ingestion, by the physiological liquid, said self-emulsionable system comprising:
- at least one active ingredient;
and the following excipients:
- a lipophilic phase;
- a surfactant having a hydrophilic/lipophilic balance (HLB) greater than 8;
- possibly a co-surfactant;
said system being obtainable by printing in 3 dimensions (3D).

2. System according to claim 1, **characterized in that** the surfactant represents at least 40% by total weight of the excipients, preferably 50%.

3. System according to one of the preceding claims, **characterized in that** the surfactant is solid at room temperature and has a melting point between 25°C and 60°C.

4. System according to one of the preceding claims, **characterized in that** the surfactant is selected from the group comprising polyethylene glycol esters, poloxamers, ethoxylated oils, ethoxylated vitamin E, sugar esters with fatty acids, and mixtures thereof.

5. System according to claim 4, **characterized in that**::
- the polyethylene glycol ester is selected from the group consisting of polyethylene glycol-32 stearate, advantageously Gélucire® 48/16 and polyoxylglycerides, advantageously Gélucire® 44/14 or Gélucire® 50/13, and/or;
- poloxamer is selected from the group consisting of poloxamer P188 and poloxamer P407, and/or;
- the ethoxylated oil is polyethylene glycol-40 hydrogenated castor oil, and/or;
- The ethoxylated vitamin E is polyethylene glycol succinate vitamin E, and/or;
- the sugar ester with fatty acids is selected from the group consisting of stearate sucrose and monoester sorbitan.

6. System according to one of the preceding claims, **characterized in that** the surfactant represents between 5% and 60% by total weight of the excipients, preferably 50%.

7. System according to one of the preceding claims, **characterized in that** the surfactant is solid at room temperature and has a melting point of between 25°C and 75°C.

8. System according to one of the preceding claims, **characterized in that** the lipophilic phase comprises at least one compound selected from the group comprising vegetable oils, triglycerides, diglycerides, monoglycerides, polyoxylglycerides, and mixtures thereof.

9. System according to one of the preceding claims, **characterized in that** it further comprises a co-solvent advantageously selected from the group consisting of ethanol and Transcutol®.

10. Method for making a solid self-emulsionable system in contact with a hydrophilic phase carried, after ingestion, by the physiological liquid, said self-emulsionable system comprising:
- at least one active ingredient;
and the following excipients:
- a lipophilic phase;
- a surfactant having a hydrophilic/lipophilic balance (HLB) greater than 8;
- optionally a co-surfactant; **characterized in that** it is implemented by printing in 3 dimensions.

11. Method according to claim 10, **characterized in that** it comprises:
- mixing and heating the at least one active ingredient and the excipients at a temperature T1, advantageously higher than the melting point of the excipient with the maximum melting point;
- Reducing the temperature of the mixture of excipients to a temperature T2, advantageously the ambient temperature;
- increasing the temperature to reach a temperature T3 higher than the melting temperature of the excipient mixture, preferably at least equal to 15°C, advantageously equalt to 15°C, above the melting point of the excipient mixture.

12. Method according to one of claims 10 or 11, **characterized in that** prior to mixing at least one active ingredient; a lipophilic phase; a surfactant having a hydrophilic/lipophilic balance (HLB) greater than 8; optionally a co-surfactant, the parameters of shape, surface/volume ratio and porosity of the desired self-emulsionable solid system obtainable by printing in 3 dimensions are determined by means of suitable software.
